# EUROPEAN PATENT SPECIFICATION

(11) **EP 1 774 011 B1**
(45) Date of publication and mention of the grant of the patent: **17.06.2015**
(21) Application number: 05787262.4
(22) Date of filing: 01.07.2005
(51) Int. Cl.: C12P 13/02, C12P 7/40, C12P 7/62, C07C 233/05, C07C 57/04

(54) **Process for preparing monomers and polymers thereof**
Verfahren zur Herstellung von Monomeren und Polymere daraus
Procédé de préparation de monomeres et de polymeres associés

(30) Priority: 19.07.2004 GB 0416101
(43) Date of publication of application: 18.04.2007
(73) Proprietor: Ciba Specialty Chemicals Water Treatments Limited, Bradford, West Yorkshire BD12 0JZ (GB)
(72) Inventor: MISTRY, Dinesh 7 Necropolis Road, West Yorkshire BD7 2PU (GB); KULLAR, Jatinder Singh, Bradford West Yorkshire_BD2 4BJ (GB)
(74) Representative: Bernhardt, Wolfgang Willy-Hans
(86) International application number: PCT/EP2005/007131
(87) International publication number: WO 2006/007957

(56) References cited:
- WO-A-98/27016
- WO-A-2005/054455
- WO-A-2005/054456
- WO-A1-97/21805
- GB-A- 2 114 118
- NAGASAWA ET AL.: "Microbial production of commodity chemicals" PURE & APPL. CHEM., vol. 67, no. 7, 1995, pages 1241-1256, XP008057259 cited in the application
- YAMADA ET AL.: "Nitrile hydratase and its application to industrial production of acrylamide" BIOSCI. BIOTECH. BIOCHEM., vol. 60, no. 9, 1996, pages 1391-1400, XP008057288 cited in the application
- HUGHES J ET AL: "APPLICATION OF WHOLE CELL RHODOCOCCAL BIOCATALYSTS IN ACRYLIC POLYMER MANUFACTURE" ANTONIE VAN LEEUWENHOEK, DORDRECHT, NL, vol. 74, 1998, pages 107-118, XP002936324 cited in the application

## Description

The present invention relates to processes for preparing ethylenically unsaturated amides from the corresponding ethylenically unsaturated nitriles.

### Technical Background

It is known to manufacture ethylenically unsaturated amides, such as acrylamide by hydration of the corresponding nitrile. US4820872 describes such a process using a black copper catalyst. It is also known to convert ethylenically unsaturated nitriles to the corresponding ethylenically unsaturated carboxylic acid by reaction of concentrated sulphuric acid with the nitrile, for instance as described in EP-A-0330474.

The enzymic catalysis of chemical reactions is well-documented in the literature. It is well known to employ biocatalysts, such as microorganisms that contain enzymes, for conducting chemical reactions, or to use enzymes that are free of microorganism It is known that various ethylenically unsaturated monomers can be prepared by converting a substrate starting material into the desired monomer by use of a biocatalyst.

Nitrile hydratase enzymes are known to catalyse the hydration of nitriles to the corresponding amides. Typically nitrile hydratase enzymes can be synthesized by a variety of microorganisms, for instance microorganisms of the genus Bacillus, Bacteridium, Micrococcus, Brevibacterium, Corynebacterium, Pseudomonas, Acinetobacter, Xanthobacter, Streptomyces, Rhizobium, Klebsiella, Enterobacter, Erwinia, Aeromonas, Citrobacter, Achromobacter, Agrobacterium, Pseudonocardia, Rhodococcus and Comomonas.

It is known to produce acrylamide and ammonium acrylate from acrylonitrile on an industrial scale using as a catalyst nitrile hydratase and nitrilase respectively. When producing these products biologically it is desirable to employ an enzyme which is capable of producing aqueous solutions of acrylamide or ammonium acrylate in high concentration and yet is not poisoned by acrylonitrile and high concentrations of acrylamide or ammonium acrylate.

Many references have described the synthesis of nitrile hydratase within microorganisms. Arnaud et al., Agric. Biol. Chem. 41: (11) 2183-2191 (1977) describes the characteristics of an enzyme they refer to as 'acetonitrilase' in Brevibacterium sp R312 which degrades acetonitrile to acetate via the amide intermediate. Asano et al., Agric. Biol. Chem. 46: (5) 1183-1189 (1982) isolated Pseudomonas chlororaphis B23 which produced nitrile hydratase to catalyse the conversion of acrylonitrile to acrylamide, generating 400 g/L acrylamide. The article by Yamada et al., Agric. Biol. Chem. 50: (11) 2859-2865 (1986) entitled, "Optimum culture conditions for production by Pseudomonas chlororaphis B23 of nitrile hydratase", considered the optimisation of the medium components of the growth medium, including the inducer added for nitrile hydratase synthesis. Methacrylamide was found to be the best inducer for this organism. Methacrylamide was included in the culture at the start of growth.

A paper by Nawaz et al., Arch. Microbiol. 156:231-238 (1991), entitled 'Metabolism of acrylonitrile by Klebsielia pneumoniae' describes the isolation and growth of the bacterium K. pneumoniae and its subsequent rapid utilisation of acrylonitrile and formation of acrylamide which was then further hydrolysed to acrylic acid. The organism was isolated using an enrichment culture technique with acrylonitrile as the sole nitrogen source at pH 7.5.

Various strains of the Rhodococcus rhodochrous species have been found to very effectively produce nitrile hydratase enzyme. EP-0 307 926 describes the culturing of Rhodococcus rhodochrous, specifically strain J1 in a culture medium that contains cobalt ions. The nitrile hydratase can be used to hydrate nitriles into amides, and in particular the conversion of 3-cyanopyridine to nicotinamide. This organism is further described in EP-0362829, which describes a method for cultivating bacteria of the species Rhodococcus rhodochrous comprising at least one of urea and cobalt ion for preparing the cells of Rhodococcus rhodochrous having nitrile hydratase activity. Specifically described is Rhodococcus rhodochrous J1.

Rhodococcus rhodochrous J1, is used commercially to manufacture acrylamide monomer from acrylonitrile and this process has been described by Nagasawa and Yamada, Pure Appl. Chem. 67: 1241-1256 (1995).

A review paper by Yamada and Kobayashi, Biosci. Biotech. Biochem 60: 1391-1400 (1996) charts the development of the biocatalysed process for the production of acrylamide monomer up to a concentration of 50%. This review describes the three generations of catalyst developed for the industrial production of acrylamide culminating with Rhodococcus rhodochrous J1, a bacterium that requires cobalt as part of the nitrile hydratase enzyme which catalyses the formation of acrylamide from acrylonitrile. The nitrile hydratase is synthesised in very high levels in the bacterium due to the presence of urea as an inducer in the culture medium

Leonova et al., Appl. Biochem. Biotechnol. 88: 231-241 (2000) entitled, "Nitrile Hydratase of Rhodococcus", describes the growth and synthesis of nitrile hydratase in Rhodococcus rhodochrous M8. The NH synthesis of this strain is induced by urea in the medium, which is also used as a nitrogen source for growth by this organism. Cobalt is also required for high nitrile hydratase activity. This literature paper mainly looks at induction and metabolic effects.

Leonova et al., Appl.. Biochem. Biotechnol. 88: 231-241 (2000) states that acrylamide is produced commercially in Russia using Rhodococcus rhodochrous M8. Russian patent 1731814 describes Rhodococcus rhodochrous strain M8.

It is also known to produce ammonium acrylate directly from acrylonitrile by the action of a nitrilase enzyme (Hughes et al (1998) Antonie van Leeuwenhoek v 74 p107-118). This article describes producing 1.3 M ammonium acrylate continuously, using immobilised Rhodococcus ruber at 30°C, the catalyst having a half life of over 47 days. The nitrilase also had a very low Km value of 30 micromoles for acrylonitrile thus the concentration of acrylonitrile in the final ammonium acrylate product was zero.

Nagasawa et al., Appl. Microbiol. Biotechnol. 34:322-324 (1990) also describe the use of the nitrilase of Rhodococcus rhodochrous J1 for the synthesis of acrylic and methacrylic acid. They looked at the effects of temperature, acrylonitrile concentration and pH conditions on the reaction.

WO 2005/054456 describes a new microorganism which is Rhodococcus rhodochrous strain 2368 (NCIMB 41164) or a mutant thereof. This microorganism can produce nitrile hydratase enzyme suitable for converting acrylonitrile to acrylamide.

WO 2005/054455 describes a new microorganism which is Dietzia natronolimnaios, NCIMB 41165, or a mutant thereof. This microorganism can produce nitrile hydratase enzyme suitable for converting acrylonitrile to acrylamide or both nitrile hydratase and amidase enzymes suitable for preparing acrylic acid, or salts thereof such as ammonium acrylate.

Acrylonitrile as it is produced, e.g., by the ammonoxidation of propylene, generally contains high levels of impurities, such as acrolein. Acrolein generally occurs as a byproduct during the manufacture of acrylonitrile. Often the amount of acrolein present in acrylonitrile will be above 2 ppm and often significantly higher than this, for instance 20 ppm and sometimes as much as 50 or 100 ppm or higher.

The ability of microorganisms to remove impurities including acrolein from waste streams has been described by Wyatt and Knowles in International Biodeterioration and Biodegradation 35 (1995) p227-248. They described the use of a mixed culture of microbes in the form of an actively growing culture to detoxify a mixed waste stream. It is expected that a live mixed culture to degrade low levels of acrylic species including acrolein would occur readily.

The use of a microorganism to detoxify a mixture of nitriles or to remove nitrile from amide mixtures has been described in WO 98/27016. The nitriles include acrylonitrile, acetonitrile and acrolein cyanohydrin as they suggest that acrolein is present in the form of acrolein cyanohydrin, thus they describe the capability of their microorganism to convert acrolein cyanohydrin to acid. The detoxification of nitriles particularly those present in waste streams, including acrolein cyanohydrin to their amide and acid counterparts is described. Also described is the use of the method of detoxification treatment to remove nitrile from an amide preparation such as acrylamide. The microorganism is multiply induced to enable the conversion of a number of different nitriles in the waste streams. However the description refers in the main to detoxification of waste streams or nitrile removal from a prepared amide solution containing ppm levels of nitriles. No mention is made of the preparation of acrylamide from an acrylonitrile substrate that may contain high levels of acrolein or of the fact that the acrolein is a known problem for the preparation of high grade acrylamide polymers and thus its removal is essential. They are mainly interested in mixtures of nitriles or nitriles/amides.

It is necessary that acrylonitrile which is used to produce acrylamide or acrylic acid is essentially pure and free from impurities such as acrolein. The presence of acrolein in acrylamide monomer or blends of monomers containing acrylamide and/or acrylic acid generally results in unwanted cross-linking of the polymer. Such cross-linking is undesirable, since in the preparation of water soluble polymers, undesirable cross-linking would result in forming at least partially insoluble polymers. Since acrolein brings about uncontrolled cross-linking, its presence in monomer mixtures containing additives used to form intentionally cross-linked polymer products can also be undesirable, because such products may be rendered too cross-linked for the particular application.

Consequently it is standard practice to remove impurities, such as acrolein, from acrylonitrile prior to the conversion to either acrylamide or acrylic acid and its salts. Typically this is done by distillation in the presence of a suitable polymerisation inhibitor such as para-methoxy phenol. Normally the levels of acrolein in acrylonitrile used for this purpose must always be less than 2 ppm, and often less than 1 ppm.

GB-A-2114118 describes a method for removing aldehyde impurities in acrylonitrile and acrylamide. The method states that aldehyde impurities, such as acrolein, in acrylonitrile and acrylamide can be removed by contacting with a weakly basic gel type polystyrene-polyamine type anion exchange resin having a styrene-divinylbenzene matrix and primary and/or secondary functional groups. The quality of acrylamide is improved which enables the production of polymers of satisfactory molecular weight for use as flocculants in water treatment and other applications.

EP-A-0110861 concerns a process for the removal of acrolein from acrylonitrile product streams. Acrolein is removed from a crude acrylonitrile product stream in a recovery column by maintaining the pH in the zone of maximum acrolein concentration of the recovery column at 5.25 to 7. Most of the acrolein exits the column through the bottom stream. High purity acrylonitrile is recovered from the top stream.

EP-A-0999207 reveals a method for removal of aldehydes from chemical manufacturing production streams using a distillative purification technique. The method describes improving purification efficiency when distilling off aldehydes such as acrolein during chemical manufacturing processes by adding a substituted aromatic amine (2-amino aniline, 3,4-dimethyl aniline and 4-ethyl aniline) prior to the distillation column.

US 5606094 describes a method for removing acrolein from a gaseous or liquid mixture by reaction with a chemical scavenger such as sodium hypochlorite, hydroxylamine, urea, thiourea and sodium bisulphate. This method particularly concerns removing acrolein from gaseous or liquid mixtures also containing acrylonitrile where the acrolein is selectively removed.

Such additional processing steps for removing acrolein are both costly and time-consuming. Consequently it would be desirable to provide a process that avoids the need to separately remove acrolein from ethylenically unsaturated nitriles, e.g. acrylonitrile.

### Disclosure of the Invention

Therefore according to the present invention we provide a process of preparing an ethylenically unsaturated amide from the corresponding ethylenically unsaturated nitrile, in which the nitrile is subjected to a hydration reaction in an aqueous medium in the presence of a biocatalyst, which biocatalyst is a microorganism of the Rhodococcus genus that is capable of producing a nitrile hydratase,
wherein the nitrile contains above 2 ppm acrolein and up to 500 ppm and the amide contains less than 2 ppm acrolein; and
wherein a step of separately removing acrolein from the ethylenically unsaturated nitrile is avoided.

### Detailed Description of the Invention

The biocatalyst may be a microorganism in the form of whole microbial cells or fractured microbial cells that contains an enzyme or enzymes capable of converting an ethylenically unsaturated nitrile to the corresponding amide. The enzyme is nitrile hydratase. The biocatalyst may be used as a fermentation broth containing the cellular material; cells or disrupted cellular material recovered by centrifuging; an aqueous suspension prepared using any suitable suspending medium such as water or physiologically compatible buffer solution. Alternatively the biocatalyst can be a purified enzyme or mixture of enzymes extracted from the microorganism.

We have found that using a biocatalyst to convert nitriles that contain acrolein to the corresponding amide, that concentrations of acrolein are significantly reduced. Typically the levels of acrolein are found to be reduced to below 2 ppm, usually below 1 ppm, and often below the level of detection. It is therefore possible to use an ethylenically unsaturated nitrile without the need for additional processing by the nitrile manufacturer to reduce the level of acrolein to less than 2 ppm.

In one aspect of the invention the process concerns the manufacture of an ethylenically unsaturated amide from the corresponding nitrile, and especially the manufacture of (meth) acrylamide from (meth) acrylonitrile. The biocatalyst is a microorganism that is capable of producing a nitrile hydratase.

The biocatalyst is a microorganism of the Rhodococcus genus, and could be of the Rhodococcus rhodochrous species. One suitable microorganism strain is Rhodococcus rhodochrous J1 as described in EP-A-0307926. A particularly suitable biocatalyst is Rhodococcus rhodochrous strain 2368 (NCIMB 41164) which is described and claimed in WO 2005/054456. The biocatalyst may be a mutant of Rhodococcus rhodochrous strain 2368 or a nitrile hydratase, obtainable from Rhodococcus rhodochrous strain 2368 or a mutant thereof.

The biocatalyst may comprise cellular material in the form of whole cells or fractured cells and optionally comprises fermentation broth. The cellular material may include any of the constituents of a microbial cell, for instance including cell wall material, cell nucleic acid material (for instance DNA or RNA), cytoplasm or proteins.

In one preferred way of carrying out the process the biocatalyst comprising of a microorganism is introduced into an aqueous medium suitable for carrying out the culturing of the microorganism. Typically a suspension of the biocatalyst, for instance whole cells of the microorganism, may be formed. A nitrile, for instance acrylonitrile or methacrylonitrile, is fed into the aqueous medium comprising the biocatalyst in such a way that the concentration of (meth) acrylonitrile in the aqueous medium is maintained at up to 6% by weight.
Nitriles such as acrylonitrile or methacrylonitrile is more preferably fed into the reaction medium and the reaction allowed to continue until the concentration of an ethylenically unsaturated monomer, either amide, for instance acrylamide or methacrylamide, or carboxylic acid, for instance acrylic acid (or salts) or methacrylic acid (or salts), reaches the desired level, in particular between 30 and 55% by weight. Most preferably the concentration is around 35-50% by weight.

The nitrile used in the process of the invention will contain above 2 ppm acrolein (calculated by weight based on total weight of nitrile) and often significantly higher than this, for instance 20 ppm and as much as 50 or 100 ppm or higher.

In another aspect the invention also includes the use of a biocatalyst which biocatalyst is a microorganism of the Rhodococcus genus that is capable of producing a nitrile hydratase for the purpose of reducing the level of acrolein in an ethylenically unsaturated monomer. We have found that the biocatalyst can be used to reduce the level of acrolein in ethylenically unsaturated monomers selected from the group consisting of (meth) acrylamide and (meth) acrylonitrile. In this aspect of the invention the biocatalyst-may include any of the preferred features described above.

A particular advantage of the present invention is that monomers obtained from (meth) acrylonitrile can be prepared conveniently without the need for removal of acrolein. Consequently, the process for producing acrylamide and acrylic acid monomers can be streamlined. In addition, it is now possible to produce these monomers directly from low purity acrylonitrile containing high levels of acrolein (greater than 2 ppm, such as at least 5 ppm, and even at least 10 ppm). The monomers produced by this process are of high-quality, and containing less than 2 ppm acrolein, and usually undetectable levels or no acrolein. Hence, polymers free from the deleterious effects of acrolein can be conveniently prepared from a monomer or monomer blend containing (meth) acrylamide and (meth) acrylic acid (or salts) that have been obtained directly from acrylonitrile that contains high levels of acrolein.

In a further aspect of the invention we provide a process for preparing a polymer of an ethylenically unsaturated amide monomer or blend comprising the ethylenically unsaturated amide monomer, which monomer has been formed from an ethylenically unsaturated nitrile, comprising the steps,
(i) contacting the ethylenically unsaturated nitrile with a biocatalyst, which biocatalyst is a microorganism of the Rhodococcus genus that is capable of producing a nitrile hydratase, to form the ethylenically unsaturated monomer,
(ii) optionally mixing the ethylenically unsaturated monomer with other monomers to form a blend, and
(iii) subjecting the ethylenically unsaturated monomer or blend to polymerisation conditions thereby forming the polymer,
   wherein the ethylenically unsaturated nitrile contains above 2 ppm acrolein and up to 500 ppm and the ethylenically unsaturated amide or carboxylic acid monomer contains less than 2 ppm acrolein,
   wherein a step of separately removing acrolein from the ethylenically unsaturated nitrile is avoided.

Preferably the ethylenically unsaturated monomer is (meth) acrylamide.

In this aspect of the invention the biocatalyst may include any of the preferred features described above. Generally the amount of acrolein present in the nitrile is as described previously.

The ethylenically unsaturated monomer can be used in the process alone to form the homopolymer or it can be mixed with other polymerisable compounds including ethylenically unsaturated monomers to form a monomer mixture that is polymerised to form a copolymer of the ethylenically unsaturated monomer. Any suitable co-monomer may be used for this purpose, preferably where the ethylenically unsaturated monomer is water-soluble. The co-monomer should desirably be water-soluble or potentially water-soluble, such as anhydrides. Typical co-monomers include (meth) acrylamide, (meth) acrylic acid (or salts), itaconic acid (or salts), maleic acid (or salts), maleic anhydride, vinyl sulfonic acid (or salts), allyl sulfonic acid (or salts), 2-acrylamido-2-methyl propane sulfonic acid (or salts), dimethyl amino ethyl (meth) acrylate (or quaternary ammonium salts), dimethyl amino propyl (meth) acrylamide (or quaternary ammonium salts), N-vinyl pyrrolidone, N-vinyl formamide, vinyl acetate, acrylonitrile, (meth) acrylic esters of C₁₋₃₀ alcohols. The salts of the above stated acid monomers may be of any suitable cation but preferably alkali metal or ammonium salts.

The process of the present invention is particular suitable for preparing high molecular weight water-soluble or water swellable polymers. The polymers may for instance be linear, branched or cross-linked. Preferably the polymers are high molecular weight substantially water-soluble that exhibit an intrinsic viscosity (IV) of at least 3 dl/g (measured using a suspended level viscometer in 1 M sodium chloride at 25°C). Usually the polymers will have intrinsic viscosities of at least 4 dl/g and generally significantly higher, for instance at least 7 or 8 dl/g. In many cases the polymers will have IV's of at least 10 or 12 dl/g and could be as high as 20 or 30 dl/g.

The water-soluble or water-swellable polymer prepared according to the process of the present invention may be cationic, anionic, non-ionic or amphoteric. It may be substantially linear or alternatively branched or cross-linked. Cross-linked or branched polymers are prepared by incorporating a branching or cross-linking agent into the monomer blend. The cross-linking or branching agent may be for instance a di- or multifunctional material that reacts with functional groups pendant on the polymer chain, for instance multivalent metal ions or amine compounds which can react with pendant carboxylic groups. Preferably, however, the cross-linking or branching agent will be a polyethylenically unsaturated compound, which becomes polymerised into two or more polymer chains. Typically such cross-linking agents include methylene-bis-acrylamide, tetra allyl ammonium chloride, triallyl amine and ethylene glycol diacrylate. The polymers may be highly crosslinked and therefore water insoluble but water swellable. Alternatively the polymer may be water soluble and either substantially linear or slightly branched, for instance prepared using less than 10 ppm cross-linking/branching monomer. In preparing cross-linked polymers, branched water-soluble polymers or linear water-soluble polymers, it is important that the monomers are free from acrolein, since this could lead to unpredictable levels of cross-linking or branching which would have deleterious effect on properties of the polymer.

Particularly preferred polymers made by the process of the invention include homopolymers or copolymers of acrylamide or methacrylamide. Desirably the copolymers include any of the above stated co-monomers but preferably it is a copolymer of acrylamide with sodium acrylate or a copolymer of acrylamide with quaternary ammonium and acid salts of dimethylaminoethyl (meth)acrylate. Especially preferred acrylamide homo or copolymers are of high molecular weight and exhibit high intrinsic viscosity as defined above.

The polymer is generally formed by subjecting the ethylenically unsaturated monomer or a monomer mixture comprising the ethylenically unsaturated monomer to polymerisation conditions. This may be achieved by heating or irradiation, for instance using ultraviolet light. Preferably polymerisation initiators are introduced into the monomer or mixture of monomers to initiate polymerisation. Desirably this may be achieved by the use of redox initiators and/or thermal initiators. Typically redox initiators include a reducing agent such as sodium sulphite, sulphur dioxide and an oxidising compound such as ammonium persulphate or a suitable peroxy compound, such as tertiary butyl hydroperoxide etc. Redox initiation may employ up to 10,000 ppm (based on weight of monomer) of each component of the redox couple. Preferably though each component of the redox couple is often less than 1000 ppm, typically in the range 1 to 100 ppm, normally in the range 4 to 50 ppm. The ratio of reducing agent to oxidizing agent may be from 10:1 to 1:10, preferably in the range 5:1 to 1:5, more preferably 2:1 to 1:2, for instance around 1:1.

Polymerisation may also be effected by employing a thermal initiatior alone or in combination with other initiator systems, for instance redox initiators. Thermal initiators would include any suitable initiator compound that releases radicals at an elevated temperature, for instance azo compounds, such as azobisisobutyronitrile (AZDN), 4,4'-azobis-(4-cyanovaleric acid) (ACVA). Typically thermal initiators are used in an amount of up 10,000 ppm, based on weight of monomer. In most cases, however, thermal initiators are used in the range 100 to 5,000 ppm preferably 200 to 2,000 ppm, usually around 1,000 ppm.

Typically an aqueous solution of water soluble monomer may be polymerised by solution or bulk polymerisation to provide an aqueous solution or gel or by reverse phase polymerisation in which an aqueous solution of monomer is suspended in a water immiscible liquid and polymerised to form polymeric beads or alternatively by emulsifying aqueous monomer into an organic liquid and then effecting polymerisation. Examples of reverse phase polymerisation are given in EP-A-150933, EP-A-102760 or EP-A-126528.

The following examples are intended to illustrate the invention, without being in any way limiting.

### Example 1

### Preparation of Acryamide

Rhodococcus rhodochrous strain 2368 (0.11 gram dry cells) and containing nitrile hydratase, is added to water (625g). The reaction mixture is heated up to 25°C with stiring.

Acrylonitrile containing 50 ppm acrolein is fed into the reactor at a rate to maintain the concentration of acrylonitrile at a maximum of 3%. After 300mins the acrylonitrile is fully converted to acrylamide at a final concentration of approximately 50%w/w. Analysis of the acrylamide shows it to be free of acrolein to below detectable limits.

The method of analysis used for low levels (below 5 ppm) of acrolein is GC-MS and for levels of acrolein above this GC-FID can be used.

### Example 2

Acrolein reduction was studied using an acrylonitrile solution containing an acrolein level of 500 ppm. Thus:
Rhodococcus rhodochrous 2368 (0.01 gram dry cells) is added to a mixture of acrylonitrile (1 gram) and water (19.0 grams) and acrolein in a 25 ml bottle. The bottle was incubated at 15°C with continuous stirring. Samples were withdrawn periodically and centrifuged prior to analysis by GC-FID for acrolein content. After 10 minutes the acrolein level in the mixture reduced from 500 ppm to below detectable limits.

### Example 3

Acrolein reduction was studied using an acrylonitrile solution containing an acrolein level of 500 ppm. Rhodococcus rhodochrous J1 (0.01 gram dry cells) is added to a mixture of acrylonitrile (1 gram) and water (19.0 grams) and acrolein in a 25 ml bottle. The bottle was incubated at 15°C with continuous stirring. Samples were withdrawn periodically and centrifuged prior to analysis by GC-FID for acrolein content. After 10 minutes the acrolein level in the mixture reduced from 500 ppm to below detectable limits.

### Example 4

Example 1 is repeated using acrylonitrile containing acrolein levels less than 2 ppm. Analysis of the acrylamide shows it to be free of acrolein.

By comparison with Example 1 we have found that the reaction rate of converting acrylonitrile to acrylamide is approximately the same when using acrylonitrile either with or without acrolein present.

High molecular weight polymer prepared using acrylamide made from acrylonitrile containing 50 ppm acrolein from Example 1 is of similar quality to high molecular weight polymers using acrylamide prepared from acrylonitrile that contained <2 ppm acrolein. The performance of the polymer as flocculant in waste water treatment applications shows no differences by varying the levels of acrolein in the acrylonitrile. The solubility and molecular weight of polymer manufactured is also suitable for use in paper making applications.

## Claims

1. A process of preparing an ethylenically unsaturated amide from the corresponding ethylenically unsaturated nitrile,
in which the nitrile is subjected to a hydration reaction in an aqueous medium in the presence of a biocatalyst, which biocatalyst is a microorganism of the Rhodococcus genus that is capable of producing a nitrile hydratase,
wherein the nitrile contains above 2 ppm acrolein and up to 500 ppm and the amide contains less than 2 ppm acrolein; and
wherein a step of separately removing acrolein from the ethylenically unsaturated nitrile is avoided.

2. A process according to claim 1, in which the biocatalyst is a microorganism of the Rhodococcus rhodochrous species.

3. A process according to claim 1 or 2, in which the biocatalyst is selected from the group consisting of a microorganism which is Rhodococcus rhodochrous strain 2368 (NCIMB 41164), and a nitrile hydratase obtainable from Rhodococcus rhodochrous strain 2368.

4. A process according to claim 1 or 2, in which the biocatalyst is selected from the group consisting of a microorganism which is Rhodococcus rhodochrous J1, and a nitrile hydratase obtainable from Rhodococcus rhodochrous J1.

5. A process according to any of claims 1 to 4, in which the biocatalyst comprises whole cells.

6. A process according to any of claims 1 to 5, in which the biocatalyst comprises fractured cellular material.

7. A process according to any of claims 1 to 6, in which the amide is (meth)acrylamide.

8. A process according to any of claims 1 to 7, in which the nitrile contains at least 10 ppm acrolein.

9. A process according to any of claims 1 to 8, in which the nitrile contains at least 20 ppm acrolein.

10. Use of a biocatalyst, which biocatalyst is a microorganism of the Rhodococcus genus that is capable of producing a nitrile hydratase, for the purpose of reducing the level of acrolein in an ethylenically unsaturated amide or nitrile monomer.

11. Use according to claim 10, in which the ethylenically unsaturated monomer is selected from the group consisting of (meth) acrylamide and (meth) acrylonitrile.

12. Use according to claim 10 or claim 11, in which the biocatalyst has any of the features defined in claims 2 to 6.

13. A process for preparing a polymer of an ethylenically unsaturated amide monomer or blend comprising the ethylenically unsaturated amide monomer, which monomer has been formed from an ethylenically unsaturated nitrile, comprising the steps,
(i) contacting the ethylenically unsaturated nitrile with a biocatalyst, which biocatalyst is a microorganism of Rhododcoccus genus that is capable of producing a nitrile hydratase, to form the ethylenically unsaturated monomer,
(ii) optionally mixing the ethylenically unsaturated monomer with other monomers to form a blend, and
(iii) subjecting the ethylenically unsaturated monomer or blend to polymerisation conditions thereby forming the polymer,
wherein the ethylenically unsaturated nitrile contains above 2 ppm and up to 500 ppm acrolein and the ethylenically unsaturated amide monomer contains less than 2 ppm acrolein, and
wherein a step of separately removing acrolein from the ethylenically unsaturated nitrile is avoided.

14. A process according to claim 13, in which the ethylenically unsaturated monomer is selected from the group consisting of (meth) acrylamide.

15. A process according to claim 13 or claim 14, in which the biocatalyst has any of the features defined in claims 2 to 6.

16. A process according to any of claims 13 to 15, in which the nitrile contains at least 10 ppm acrolein.

17. A process according to any of claims 13 to 16, in which the nitrile contains at least 20 ppm acrolein.

## Patentansprüche

1. Verfahren zum Herstellen eines ethylenisch ungesättigten Amids aus dem entsprechenden ethylenisch ungesättigten Nitril,
wobei das Nitril einer Hydratationsreaktion in einem wässrigen Medium in Gegenwart eines Biokatalysators unterworfen wird, welcher Biokatalysator ein Mikroorganismus der Gattung Rhodococcus ist, der fähig ist, eine Nitrilhydratase herzustellen,
wobei das Nitril mehr als 2 ppm und bis zu 500 ppm Acrolein enthält und das Amid weniger als 2 ppm Acrolein enthält; und
wobei ein Schritt des gesonderten Entfernens von Acrolein von dem ethylenisch ungesättigten Nitril vermieden wird.

2. Verfahren gemäß Anspruch 1, wobei der Biokatalysator ein Mikroorganismus der Spezies Rhodococcus rhodochrous ist.

3. Verfahren gemäß Anspruch 1 oder 2, wobei der Biokatalysator ausgewählt ist aus der Gruppe bestehend aus einem Mikroorganismus, der Rhodococcus rhodochrous Stamm 2368 (NCIMB 41164) ist, und einer Nitrilhydratase, erhältlich aus Rhodococcus rhodochrous Stamm 2368.

4. Verfahren gemäß Anspruch 1 oder 2, wobei der Biokatalysator ausgewählt ist aus der Gruppe bestehend aus einem Mikroorganismus, der Rhodococcus rhodochrous J1 ist, und einer Nitrilhydratase, erhältlich aus Rhodococcus rhodochrous J1.

5. Verfahren gemäß einem der Ansprüche 1 bis 4, wobei der Biokatalysator Gesamtzellen umfasst.

6. Verfahren gemäß einem der Ansprüche 1 bis 5, wobei der Biokatalysator aufgebrochenes Zellmaterial umfasst.

7. Verfahren gemäß einem der Ansprüche 1 bis 6, wobei das Amid (Meth)acrylamid ist.

8. Verfahren gemäß einem der Ansprüche 1 bis 7, wobei das Nitril wenigstens 10 ppm Acrolein enthält.

9. Verfahren gemäß einem der Ansprüche 1 bis 8, wobei das Nitril wenigstens 20 ppm Acrolein enthält.

10. Verwendung eines Biokatalysators, welcher Biokatalysator ein Mikroorganismus der Gattung Rhodococcus ist, der fähig ist, eine Nitrilhydratase herzustellen, für den Zweck der Verringerung der Menge an Acrolein in einem ethylenisch ungesättigten Amid- oder Nitrilmonomer.

11. Verwendung gemäß Anspruch 10, wobei das ethylenisch ungesättigte Monomer ausgewählt ist aus der Gruppe bestehend aus (Meth)acrylamid und (Meth)acrylnitril.

12. Verwendung gemäß Anspruch 10 oder Anspruch 11, wobei der Biokatalysator eines der in den Ansprüchen 2 bis 6 definierten Merkmale aufweist.

13. Verfahren zum Herstellen eines Polymers eines ethylenisch ungesättigten Amidmonomers oder eines Gemischs, umfassend das ethylenisch ungesättigte Amidmonomer, welches Monomer aus einem ethylenisch ungesättigten Nitril gebildet worden ist, umfassend die Schritte
(i) Inkontaktbringen des ethylenisch ungesättigten Nitrils mit einem Biokatalysator, welcher Biokatalysator ein Mikroorganismus der Gattung Rhodococcus ist, der fähig ist, eine Nitrilhydratase herzustellen, um das ethylenisch ungesättigte Monomer zu bilden,
(ii) gegebenenfalls Mischen des ethylenisch ungesättigten Monomers mit anderen Monomeren, um ein Gemisch zu bilden, und
(iii) Unterwerfen des ethylenisch ungesättigten Monomers oder des Gemischs an Polymerisationsbedingungen, um so das Polymer zu bilden,
wobei das ethylenisch ungesättigte Nitril mehr als 2 ppm und bis zu 500 ppm Acrolein enthält und das ethylenisch ungesättigte Amidmonomer weniger als 2 ppm Acrolein enthält, und
wobei ein Schritt des gesonderten Entfernens von Acrolein von dem ethylenisch ungesättigten Nitril vermieden wird.

14. Verfahren gemäß Anspruch 13, wobei das ethylenisch ungesättigte Monomer ausgewählt ist aus der Gruppe bestehend aus (Meth)acrylamid.

15. Verfahren gemäß Anspruch 13 oder Anspruch 14, wobei der Biokatalysator eines der in den Ansprüchen 2 bis 6 definierten Merkmale aufweist.

16. Verfahren gemäß einem der Ansprüche 13 bis 15, wobei das Nitril wenigstens 10 ppm Acrolein enthält.

17. Verfahren gemäß einem der Ansprüche 13 bis 16, wobei das Nitril wenigstens 20 ppm Acrolein enthält.

## Revendications

1. Procédé de préparation d'un amide éthyléniquement insaturé à partir du nitrile éthyléniquement insaturé correspondant,
dans lequel le nitrile est soumis à une réaction d'hydratation dans un milieu aqueux en présence d'un biocatalyseur, lequel biocatalyseur est un microorganisme du genre *Rhodococcus* qui est capable de produire une nitrile hydratase,
où le nitrile contient plus de 2 ppm d'acroléine et jusqu'à 500 ppm et l'amide contient moins de 2 ppm d'acroléine ; et
dans lequel on évite une étape consistant à éliminer séparément l'acroléine à partir du nitrile éthyléniquement insaturé.

2. Procédé selon la revendication 1, dans lequel le biocatalyseur est un microorganisme de l'espèce *Rhodococcus rhodochrous.*

3. Procédé selon la revendication 1 ou 2, dans lequel le biocatalyseur est choisi dans le groupe constitué par un microorganisme qui est la souche 2368 de *Rhodococcus rhodochrous* (NCIMB 41164), et une nitrile hydratase pouvant être obtenue à partir de la souche 2368 de *Rhodococcus rhodochrous.*

4. Procédé selon la revendication 1 ou 2, dans lequel le biocatalyseur est choisi dans le groupe constitué par un microorganisme qui est *Rhodococcus rhodochrous* J1, et une nitrile hydratase pouvant être obtenue à partir de *Rhodococcus rhodochrous* J1.

5. Procédé selon l'une quelconque des revendications 1 à 4, dans lequel le biocatalyseur comprend des cellules entières.

6. Procédé selon l'une quelconque des revendications 1 à 5, dans lequel le biocatalyseur comprend du matériel cellulaire fracturé.

7. Procédé selon l'une quelconque des revendications 1 à 6, dans lequel l'amide est le (méth)acrylamide.

8. Procédé selon l'une quelconque des revendications 1 à 7, dans lequel le nitrile contient au moins 10 ppm d'acroléine.

9. Procédé selon l'une quelconque des revendications 1 à 8, dans lequel le nitrile contient au moins 20 ppm d'acroléine.

10. Utilisation d'un biocatalyseur, lequel biocatalyseur est un microorganisme du genre *Rhodococcus* qui est capable de produire une nitrile hydratase, aux fins de réduire le taux d'acroléine dans un monomère d'amide ou de nitrile éthyléniquement insaturé.

11. Utilisation selon la revendication 10, dans laquelle le monomère éthyléniquement insaturé est choisi dans le groupe constitué par le (méth)acrylamide et le (méth)acrylonitrile.

12. Utilisation selon la revendication 10 ou la revendication 11, dans laquelle le biocatalyseur possède l'une quelconque parmi les caractéristiques définies selon les revendications 2 à 6.

13. Procédé de préparation d'un polymère d'un monomère d'amide éthyléniquement insaturé ou d'un mélange comprenant le monomère d'amide éthyléniquement insaturé, lequel monomère a été formé à partir d'un nitrile éthyléniquement insaturé, comprenant les étapes
(i) de mise en contact du nitrile éthyléniquement insaturé avec un biocatalyseur, lequel biocatalyseur étant un microorganisme du genre *Rhodococcus* qui est capable de produire une nitrile hydratase, pour former le monomère éthyléniquement insaturé,
(ii) de mélange éventuel du monomère éthyléniquement insaturé avec d'autres monomères pour former un mélange, et
(iii) de soumission du monomère éthyléniquement insaturé ou du mélange à des conditions de polymérisation, formant ainsi le polymère,
où le nitrile éthyléniquement insaturé contient plus de 2 ppm et jusqu'à 500 ppm d'acroléine et le monomère d'amide éthyléniquement insaturé contient moins de 2 ppm d'acroléine ; et
dans lequel on évite une étape consistant à éliminer séparément l'acroléine à partir du nitrile éthyléniquement insaturé.

14. Procédé selon la revendication 13, dans lequel le monomère éthyléniquement insaturé est choisi dans le groupe constitué par le (méth)acrylamide.

15. Procédé selon la revendication 13 ou la revendication 14, dans lequel le biocatalyseur possède l'une quelconque parmi les caractéristiques définies selon les revendications 2 à 6.

16. Procédé selon l'une quelconque des revendications 13 à 15, dans lequel le nitrile contient au moins 10 ppm d'acroléine.

17. Procédé selon l'une quelconque des revendications 13 à 16, dans lequel le nitrile contient au moins 20 ppm d'acroléine.
